# EUROPÄISCHE PATENTSCHRIFT

(11) **EP 0 711 754 B1**
(45) Veröffentlichungstag und Bekanntmachung des Hinweises auf die Patenterteilung: **10.03.1999**
(21) Anmeldenummer: 95117248.5
(22) Anmeldetag: 02.11.1995
(51) Int. Cl.: C07C 323/09, C07C 319/14

(54) **Verfahren zur Herstellung von 2-Chlor-6-nitrophenyl-alkylsulfanen und neue 2-Chlor-6-nitrophenyl-alkyl-sulfane**
Process for preparing 2-chloro-6-nitrophenylalkylsulfides as well as novel 2-chloro-6-nitrophenyl alkylsulfides
Procédé pour la préparation de 2-chloro-6-nitrophényle-alkylesulfures ainsi que nouveaux 2-chloro-6-nitrophényle-alkyle-sulfures

(30) Priorität: 14.11.1994 DE 4440595
(43) Veröffentlichungstag der Anmeldung: 15.05.1996
(73) Patentinhaber: BAYER AG, 51368 Leverkusen (DE)
(72) Erfinder: Fiege, Helmut, Dr., D-51373 Leverkusen (DE); Hagedorn, Ferdinand, Dr., D-51381 Leverkusen (DE)

(56) Entgegenhaltungen:
- J. CHEM. SOC. (B), 1966 Seiten 260-266, GRUNDON ET AL. 'Proximity Effects in Diaryl Derivatives. Part IV.'
- BULL. SOC. CHIM. FR., 1951 Seiten 621-626,
- HOUBEN-WEYL 'Band E11: Organische Schwefel-Verbindungen' 1985 , GEORG THIEME VERLAG * Seite 175 *

## Beschreibung

Die Erfindung betrifft ein Verfahren zur Herstellung von 2-Chlor-6-nitrophenyl-alkylsulfanen durch Umsetzung der zugrundeliegenden 2,3-Dichlor-nitrobenzole mit Mercaptanen in Gegenwart von Base und in weiterer Gegenwart eines Phasentransferkatalysators. Die Erfindung betrifft weiterhin neue 2-Chlor-6-nitrophenyl-alkylsulfane. Sulfane dieser Art finden beispielsweise Verwendung zur Herstellung von Wirkstoffen für den Pflanzenschutz und als Zwischenprodukte für Farbstoffe (Phosphorus and Sulfur 7 (1979), 143-148; JP 57/58662 (1982)).

Es ist bekannt, Chlornitrobenzole mit aktivierten Chloratomen in alkoholischer Lösung oder Suspension in Gegenwart von Natronlauge oder Kaliumcarbonat durch Einleiten von Methanthiol in die entsprechenden Aryl-methyl-sulfane zu überführen (Houben-Weyl, Bd. E 11 (1985), S. 175). Die Ausbeuten erreichen nach dieser Methode jedoch nur 52-78 % der theoretischen Ausbeute. Die Reaktion von 2,3-Dichlor-nitrobenzol mit Benzylmercaptan in Gegenwart einer Base lieferte 2-Chlor-6-nitrophenyl-benzylsulfan mit nur 56 % Ausbeute (Bull. Soc. Chem. Fr.) 1951, 621-626).

Es bestand ein Bedürfnis, einen auch technisch geeigneten Zugang zu diesen Verbindungen zu finden. Hierbei war nicht auszuschließen, daß auf Grund der reduzierenden Wirkung der Mercapto-Verbindung eine andersartige als die gewünschte Reaktion, beispielsweise die Reduktion der Nitrogruppe eintreten konnte. So konnte die Bildung des zugehörigen Anilins erwartet werden. Weiterhin mußte mit nukleophilen Verdrängungsreaktionen durch das Mercaptid auch an anderen Substituenten des Benzolkerns gerechnet werden, die eine solche Verdrängung erlauben (J. Org. Chem. 43, 2048-2055 (1978)). Die Folge solcher Nebenreaktionen wäre die Bildung gegebenenfalls schwerer abtrennbarer Nebenprodukte und in jedem Falle die Minderung der Ausbeute, wie das im Falle der oben angegebenen Zeitschriftenzitate anzunehmen ist.

Die Erfindung betrifft ein Verfahren zur Herstellung von 2-Chlor-6-nitrophenyl-alkylsulfanen der Formel 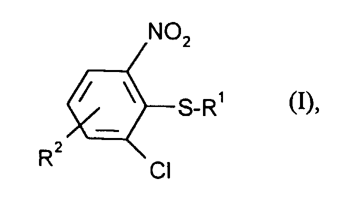 in der
- R¹: für geradkettiges oder verzweigtes C₁-C₁₈-Alkyl, C₃-C₈-Cycloalkyl oder C₇-C₁₀-Aralkyl steht und
- R²: Wasserstoff, geradkettiges oder verzweigtes C₁-C₄-Alkyl, geradkettiges oder verzweigtes C₁-C₄-Alkoxy oder Benzyl bedeutet,
das dadurch gekennzeichnet ist, daß man 2,3- Dichlor-nitrobenzole der Formel 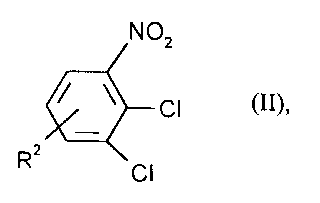 mit 0,9-2 Mol, bevorzugt 1-1,5 Mol eines Mercaptans der Formel

HS-R¹ (III),

wobei
- R¹ und R²: die obigen Bedeutungen haben,
in Gegenwart von 1-1,2, bevorzugt 1-1,1 Äquivalenten einer Base pro Mol Mercaptan und in weiterer Gegenwart eines Phasentransferkatalysators bei einer Temperatur von 0-100°C, bevorzugt 20-80°C in wäßrigem oder wäßrig-organischem Medium umsetzt.

Die Erfindung betrifft ferner neue 2-Chlor-6-nitrophenyl-alkylsulfane im Umfang der Formel (I) mit der Maßgabe, daß für den Fall, daß R² Wasserstoff bedeutet, R¹ nicht die Bedeutung von Methyl, Ethyl oder Benzyl annimmt.

Geradkettiges oder verzweigtes C₁-C₄-Alkyl und weiterhin C₁-C₁₈-Alkyl ist beispielsweise Methyl, Ethyl, Propyl, Isopropyl, Butyl, Isobutyl, tert.-Butyl, die isomeren Amyle, die isomeren Hexyle, Octyle, Decyle, Dodecyle, Heptadecyle und Octadecyle.

C₃-C₈-Cycloalkyl ist beispielsweise Cyclopropyl, Cyclobutyl, Cyclopentyl, Cyclohexyl, Cycloheptyl, Cyclooctyl, Methyl-cyclopropyl, Dimethyl-cyclopropyl, Methyl-cyclopentyl, Dimethyl-cyclopentyl, Methyl-cyclohexyl, Dimethyl-cyclohexyl, bevorzugt Cyclopropyl, Cyclopentyl, Cyclohexyl oder deren Monomethyl- bzw. Dimethyl-Derivate.

C₇-C₁₀-Aralkyl ist beispielsweise Benzyl, α- bzw. β-Phenylethyl, Phenylpropyl oder Phenylbutyl, bevorzugt Benzyl oder Phenyl-ethyl, besonders bevorzugt Benzyl.

Geradkettiges oder verzweigtes C₁-C₄-Alkoxy ist beispielsweise Methoxy, Ethoxy, Propoxy, Isopropoxy, Butoxy oder Isobutoxy.

In bevorzugter Weise wird mit einem Mercaptan der Formel

HS-R¹¹ (IV),

umgesetzt, worin
- R¹¹: für geradkettiges oder verzweigtes C₁-C₁₂-Alkyl, nicht substituiertes, Methyl-substituiertes oder Dimethyl-substituiertes Cyclopropyl, Cyclopentyl oder Cyclohexyl oder C₇-C₈-Aralkyl steht.

In besonders bevorzugter Weise wird mit einem Mercaptan der Formel

HS-R²¹ (V),

umgesetzt, worin
- R²¹: für geradkettiges oder verzweigtes C₁-C₄-Alkyl, Cyclopropyl, Cyclohexyl oder Benzyl steht.

In weiterhin bevorzugter Weise wird ein 2,3-Dichlor-nitrobenzol der Formel 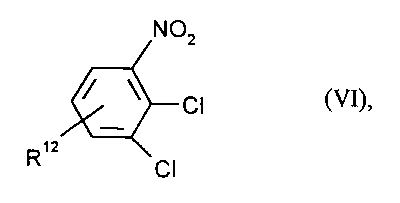 umgesetzt, worin
- R¹²: Wasserstoff, Methyl oder Methoxy bedeutet.

In weiterhin besonders bevorzugter Weise wird 2,3-Dichlor-nitrobenzol umgesetzt.

Anhand der zuletzt genannten Verbindung kann das erfindungsgemäße Verfahren formelmäßig wie folgt dargestellt werden:

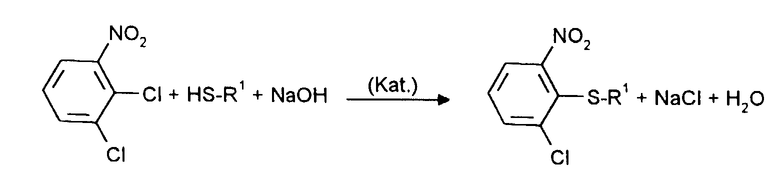

Im erfindungsgemäßen Verfahren wird je 1 Mol Dichlor-nitrobenzol (II) mit je 0,9 bis 2 Mol, bevorzugt je 1 bis 1,5 Mol Mercaptan (III) umgesetzt.

Wichtige Alkylmercaptane für das erfindungsgemäße Verfahren sind: Methylmercaptan, Ethylmercaptan, Isopropylmercaptan, tert.-Butylmercaptan, Cyclohexylmercaptan und Benzylmercaptan. Die Mercaptane sind handelsübliche Stoffe.

Die einzusetzenden Dichlor-nitrobenzole sind gleichfalls industriell zugängliche Stoffe. So steht beispielsweise 2,3-Dichlor-nitrobenzol aus der Nitrierung von o-Dichlorbenzol zur Verfügung.

Das erfindungsgemäße Verfahren wird in Gegenwart von 1-1,2, bevorzugt 1-1,1 Äquivalent einer Base pro Mol Mercaptan durchgeführt. Als geeignete Base seien genannt: Die Hydroxide und Carbonate von Alkali- und Erdalkalimetallen. Schwerlösliche oder nahezu unlösliche Verbindungen dieser Art, wie beispielsweise Erdalkalicarbonate können in suspendierter Form vorliegen und ohne Schwierigkeiten in die Reaktion eingreifen. Als weitere Basen kommen tertiäre Amine mit Alkyl- oder Benzylgruppen und mit insgesamt 3-15 C-Atomen in Betracht. Bevorzugte Basen sind beispielsweise Natronlauge, Kalilauge, Natriumcarbonat, Kaliumcarbonat, Triethylamin oder N,N-Dimethyl-benzylamin.

Als Phasentransferkatalysatoren kommen beispielsweise quarternäre Ammonium- und Phosphoniumsalze der Formeln (I) und (II) in Frage 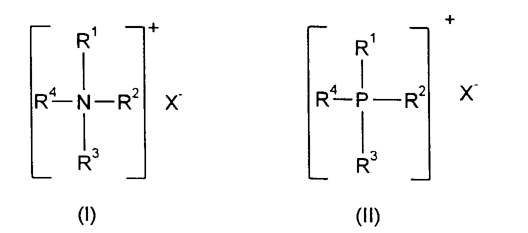 in denen
- R¹ bis R⁴: gleich oder verschieden sind und jeweils für eine C₁-C₁₆-Alkylgruppe, die gegebenenfalls mit einer Hydroxygruppe substituiert sein kann, eine C₆-C₁₀-Arylgruppe, eine C₇-C₁₁-Aralkylgruppe oder einer C₅-C₇-Cycloalkylgruppe stehen, wobei zwei der Reste R¹ bis R⁴ gemeinsam auch einen Ring mit 5 bis 7 C-Atomen bilden können und
- X: für ein Halogen, einen Bisulfat-Rest oder eine Hydroxygruppe steht.

Vorzugsweise sind R¹ bis R⁴ gleich oder verschieden und stehen jeweils für Methyl, Ethyl, n-Propyl, i-Propyl, n-Butyl, i-Butyl, t-Butyl oder Benzyl. Vorzugsweise steht X für Chlor oder Brom.

Besonders bevorzugte Phasentransferkatalysatoren sind Tetra-n-butyl-ammoniumbromid und Benzyltriethylammoniumchlorid, die besonders gut zugänglich sind.

Der Phasentransferkatalysator kann beispielsweise in einer Menge von 0,001 bis 0,2 Moläquivaaalenten, bezogen auf 2,3-Dichlor-nitrobenzol, eingesetzt werden. Vorzugsweise liegt diese Menge bei 0,01 bis 0,1 Moläquivalenten.

Das erfindungsgemäße Verfahren wird im Temperaturbereich von 0-100°C, bevorzugt 20-80°C, durchgeführt. Der Druck über dem Reaktionssystem ist nicht kritisch, so daß bevorzugt bei Normaldruck gearbeitet wird. Ein Arbeiten unter mäßig erhöhtem Druck ist lediglich erforderlich, um niedrigsiedende Reaktionsteilnehmer im flüssigen Aggregatzustand zu halten. Insofern kommt ein Druckbereich von 1-8, bevorzugt 1-5 bar in Frage.

Das erfindungsgemäße Verfahren kann in verschiedenen Varianten ausgeübt werden. So kann das Dichlor-nitrobenzol als Schmelze in die wäßrige Lösung eines Alkyl-mercaptids (gebildet durch eine der oben genannten Basen) bei gleichzeitiger Gegenwart eines Phasentransferkatalysators eingetragen werden und im gewählten Temperaturbereich umgesetzt werden.

Bevorzugt wird die Schmelze des Dichlor-nitrobenzols als Suspension in Wasser bei Anwesenheit eines Phasentransferkatalysators vorgeleget und eine wäßrige Alkylmercaptid-Lösung zugesetzt.

Weitere Ausführungsformen des Verfahrens bestehen darin, das Dichlor-nitrobenzol in einem organischen Lösungsmittel zu lösen und in der oben genannten Weise als Lösung in vorgelegtes Alkylmercaptid bei Anwesenheit des Phasentransferkatalysators einzutragen oder umgekehrt die Lösung vorzulegen und bei Anwesenheit des Phasentransferkatalysators das Alkylmercaptid in Form einer wäßrigen Lösung zuzusetzen.

Als Lösungsmittel für das Dichlor-nitrobenzol eignen sich ein C₁-C₆-Alkanol, ein C₃-C₆-Keton, ein C₆-C₁₂-Kohlenwasserstoff der (cyclo)aliphatischen oder aromatischen Reihe, ein C₁-C₈-Halogen-Kohlenwasserstoff der (cyclo)aliphatischen oder aromatischen Reihe sowie offenkettige oder cyclische Peralkyl-Carbonsäureamide mit 3-8 C-Atomen. Bevorzugte und technisch wichtige Lösungsmittel sind:
Methanol, Ethanol, i-Propanol, Aceton, Methylenchlorid, Chlorbenzol, Toluol, Xylol, Dimethylformamid oder N-Methyl-pyrrolidon.

Eine weitere Variante ist das Eintragen einer Schmelze oder einer Lösung des Dichlor-nitrobenzols in einem der genannten Lösungsmittel in vorgelegtes Alkylmercaptid in Gegenwart des Phasentransferkatalysators. Von diesen Möglichkeiten ist wiederum das Arbeiten mit einer Schmelze des Dichlor-nitrobenzols besonders bevorzugt, weil hierbei auf ein organisches Lösungsmittel mit der Notwendigkeit zur Recyclisierung und mit dem Vorteil des Verzichts auf arbeitshygienische Sicherheitsmaßnahmen in Wasser allein gearbeitet werden kann.

Wegen der Oxidationsanfälligkeit von Mercaptanen gegenüber Luft und anderen Oxidationsmitteln ist es zweckmäßig, das Mercaptan unter Schutzgas zunächst mit einer Base umzusetzen. Dabei leitet man - insbesondere bei gasförmigen oder niedrigsiedenden Mercaptanen - die Mercaptoverbindung unter die Oberfläche der Lösung oder Suspension der Base ein.

Die erfindungsgemäß hergestellten Chlor-nitrophenyl-alkylsulfane fallen bei der Reaktion als wasserunlösliche Substanzen an. Bei Temperaturen oberhalb ihres Schmelzpunktes können solche Verbindungen als Schmelze von der wäßrigen Salzlösung abgetrennt und durch Ausrühren mit heißem Wasser von Salzen befreit werden. Nach dem Ausrühren mit heißem Wasser zur Entfernung von Salzen (z.B. von Natriumchlorid) kann beispielsweise das rohe Sulfan in eine erhitzte Alkohol-Wasser-Mischung eingetragen und durch Abkühlen zur Kristallisation gebracht werden. Nach diesem Umlösevorgang besitzt das jeweilige Produkt in vielen Fällen bereits einen Gehalt von mehr 99 %. Diese hohen Reinheiten, verbunden mit hohen Selektivitäten zum gewünschten Produkt, sind insofern erstaunlich, als zu erwarten gewesen wäre, daß der Nitro-Substituent bei einem höheren Angebot an Mercaptid ebenso wie das benachbarte Chloratom einen Austausch unter Bildung einer zweiten Sulfangruppe eingehen würde. Daß ein solcher Vorgang grundsätzlich zu erwarten ist, konnte bei eigenen Versuchen nachgewiesen werden.

Hierbei ist ferner zu berücksichtigen, daß bei der bevorzugten Variante des erfindungsgemäßen Verfahrens durch Zugabe von (II) in vorgelegtes (III) zu Beginn der Reaktion ein großer Überschuß an (III) gegenüber (II) vorliegt. Ferner ist aus J. Soc. Chem. Ind. 1927, 435 T die Reduktion der Nitrogruppe im 4-Chlornitrobenzol durch Methylthiol zu 4,4'-Dichlor-azoxybenzol und schließlich zu 4-Chloranilin bekannt.

Die erfindungsgemäß herstellbaren Chlorphenyl-alkylsulfane stellen wertvolle Zwischenprodukte zur Herstellung von Farbstoffen und besonders von Wirkstoffen, beispielsweise im Bereich des Pflanzenschutzes dar. Beispielsweise seien genannt: 2-Chlor-6-nitrophenyl-methylsulfan, 2-Chlor-6-nitrophenyl-ethylsulfan, 2-Chlor-6-nitrophenyl-isopropyl-sulfan, 2-Chlor-6-nitrophenyl-benzyl-sulfan, 2-Chlor-6-nitrophenyl-cyclohexyl-sulfan.

### Beispiele

### Beispiel 1

In eine aus 21,4 g (0,525 Mol) Natriumhydroxid und 150 ml Wasser hergestellte Lösung tropfte man durch ein getauchtes Rohr unter Rühren 40,8 g (0,525 Mol) Isopropylmercaptan, 98 %ig, innerhalb 30 min bei -3 bis 0°C. Nach Zugabe von 2,5 g Tetrabutylammoniumbromid erhitzte man die Mercaptidlösung auf 65°C und tropfte bei dieser Temperatur 97,0 g aufgeschmolzenes 2,3-Dichlor-nitrobenzol, 99,4 %ig (0,5 Mol), innerhalb von 45 min zu. Das Gemisch wurde 4 h bei 65°C nachgerührt. Die überstehende wäßrige Salzlösung wurde mit wenig wäßriger Salzsäure neutralisiert und von der Produktphase abgetrennt. Danach rührte man letztere mit heißem Wasser, trennte die Phasen und löste das flüssige Produkt 115,5 g, 97,6 %ig = 97,4 % der theoretischen Ausbeute in einer heißen Lösung von Isopropanol/Wasser, aus der sich beim Abkühlen das Produkt in beigefarbenen Kristallen abschied.
Gehalt: 99,4 % (GC), Fp. 65-66°C

### Beispiel 2

In eine Lösung von 97,0 g 2,3-Dichlor-nitrobenzol (0,5 Mol) in 150 ml Methanol trug man bei 60°C eine Lösung von 42,7 g (0,55) Mol Isopropylmercaptan in wäßriger Natronlauge, hergestellt aus 22,5 g (0,5625 Mol) Natriumhydroxid in 150 ml Wasser, innerhalb von 60 min ein. Das Gemisch wurde 4 h bei 60°C nachgerührt. Anschließend destillierte man nach Neutralisation das Lösungsmittel ab, trennte das Produkt von der wäßrigen Salzlösung, wusch es mit heißem Wasser und isolierte das rohe Produkt durch Absaugen. Nach dem Trocknen erhielt man das Produkt mit einer Rohausbeute von 118,6 g. Der Gehalt betrug nach GC-Analyse 96,3 %, Ausbeute: 98,6 % der theoretischen Ausbeute. Durch Umlösen aus Isopropanol/Wasser erhielt man eine 99,4 %ige Substanz.

### Beispiel 3

In eine Lösung von 97,0 g 2,3-Dichlor-nitrobenzol (0,5 mol) und 2,5 g Tetrabutylammoniumbromid in 150 ml Toluol tropfte man bei 60°C eine Lösung von 40,8 g (0,52 Mol) Isopropylmercaptan in wäßriger Natronlauge, hergestellt aus 21,4 g (0,535 Mol) Natriumhydroxid in 150 ml Wasser, innerhalb von 55 min. Das Gemisch wurde 2 h bei 60°C nachgerührt. Danach destillierte man das Lösungsmittel mit Wasserdampf ab, trennte das Produkt von der wäßrigen Salzlösung, wusch es mit heißem Wasser, trennte es vom Wasser und isolierte nach dem Trocknen ein 97,1 %iges Produkt (GC), Ausbeute: 118 g = 97 % der theoretischen Ausbeute.

### Beispiel 4

In eine aus 21,4 g (0,525 Mol) Natriumhydroxid und 150 ml Wasser hergestellte Natronlauge tropfte man unter Rühren durch ein getauchtes Rohr 40,8 g (0,252 Mol) Isopropylmercaptan, 98 %ig, innerhalb 30 min bei -3°C. Die erhaltene Natrium-isopropylmercaptid-Lösung füllte man in einen Tropfrichter und tropfte sie unter Rühren innerhalb 55 min zu einer auf 60 bis 65°C erhitzten Suspension von 97,0 g (0,5 Mol) geschmolzenem 2,3-Dichlor-nitrobenzol und 2,5 g Tetrabutylammoniumbromid in 100 ml Wasser. Anschließend rührte man das Gemisch 3 h bei 60°C. Nach dem Abkühlen auf Raumtemperatur wurde das kristalline Produkt abgesaugt, mit Wasser gewaschen und getrocknet. Man erhielt 117,3 g einer 98,2 %igen (GC) kristallinen Rohsubstanz, entsprechend einer Ausbeute von 99,4 % der theoretischen Ausbeute. Nach Umkristallisieren aus Isopropanol/Wasser isolierte man ein 99,5 %iges (GC) Produkt, Fp. 66°C.

### Beispiel 5

In einem 250 ml Rührkolben wurden 10,7 g Natriumhydroxid (0,263 mol) in 75 ml Wasser gelöst. In diese Lösung tropfte man unter Rühren bei 10°C 32,9 g = 39,1 ml (0,263 mol) Benzylmercaptan (99 %ig). Die erhaltene Lösung tropfte man unter Rühren innerhalb 40 min. bei 60°C zu einer Suspension von 48,5 g (0,25 mol) 2,3-Dichlor-nitrobenzol (99,4 %ig) und 1,3 g Tetra-n-butylammoniumbromid in 50 ml Wasser und rührte das Gemisch 4 Stunden nach. Nach dem Abkühlen saugte man das kristalline Produkt ab, wusch es mit Wasser und trocknete es. Ausbeute roh:

| 72,7 g (theoretisch: 69,9 g, rein) Fp. 66-68°C | | |
|---|---|---|
| GC-Analyse | 93,9 % | 2-Chlor-6-nitrophenyl-thiobenzylether, |
| | 4,6 % | 2,3-Bis-(benzylmercapto)-chlorbenzol. |

### Beispiel 6

In einem 250 ml Rührkolben kühlte man eine Lösung von 10,7 g (0,263 mol) Natriumhydroxid und 75 ml Wasser auf 10°C und tropfte aus einem Tropftrichter unter Rühren 31,5 g (0,263 mol) Cyclohexylmercaptan (97 %ig) zu. Die erhaltene Emulsion (ein geringer Anteil blieb ungelöst) wurde bei 60°C in eine Suspension von 48,5 g (0,25 mol) 2,3-Dichlor-nitrobenzol (99,4 %ig) und 1,3 g Tetra-n-butylammoniumbromid in 50 ml Wasser innerhalb 40 min unter kräftigem Rühren eingetropft. Das Gemisch wurde 4 Stunden bei 60°C nachgeführt, abgekühlt und über eine Nutsche abgesaugt, das kristalline Produkt mit Wasser gewaschen und getrocknet. Ausbeute: 68,8 g roh, (theoretisch 67,9 g, rein), 97,8 %ig nach GC-Analyse, Fp. 74-76°C.

### Vergleichsbeispiel (NO₂-Verdrängung)

Man stellte eine Natriumbenzylmercaptidlösung durch Lösen von 20,4 g Natriumhydroxid, 98 %ig, (0,5 mol) in 175 ml Wasser und Zutropfen von 62,6 g (0,5 mol) Benzylmercaptan (99 %ig) her. Diese Lösung tropfte man innerhalb einer Stunde zu einer Suspension von 58,5 g (0,25 mol) 2,3-Dichlor-nitrobenzol (99,4 %ig) und 1,3 g Tetrabutylammoniumbromid in 50 ml Wasser bei 60°C unter kräftigem Rühren. Durch GC-Analysen von Proben des Reaktionsgemisches in Zeitabständen von 4 Stunden wurde das Fortschreiten der Umsetzung beobachtet:

Nach 4 Stunden bei 60°C: 37,4 % 2-Chlor-6-nitrophenyl-thiobenzylether (monosubst. Prod.) und 51,4 % 2,3-Bis-(benzylmercapto)-chlorbenzol (disubst. Prod).

Nach weiteren 4 Stunden bei 70°C: 21,1 % monosubst. Prod. und 65,3 % disubst. Prod.

Nach weiteren 4 Stunden bei 80°C: 12,7 % monosubst. Prod. und 72,0 % disubst. Prod.

Das Reaktionsgemisch wurde abgekühlt, mit 250 ml Toluol extrahiert und eingeengt, Rückstand: 92 g. Durch Umkristallisieren aus Methanol erhielt man eine 92,7 %ige Substanz (2 % monosubst. Prod.). Durch nochmalige Umkristallisation aus Methanol fiel ein 97,6 %iges Produkt vom Fp. 86-88°C an.

## Patentansprüche

1. Verfahren zur Herstellung von 2-Chlor-6-nitrophenyl-alkylsulfanen der Formel 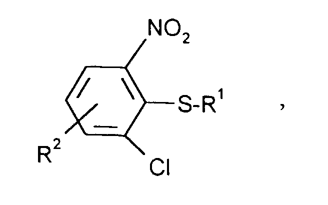 in der
R¹ für geradkettiges oder verzweigtes C₁-C₁₈-Alkyl, C₃-C₈-Cycloalkyl oder C₇-C₁₀-Aralkyl steht und
R² Wasserstoff, geradkettiges oder verzweigtes C₁-C₄-Alkyl, geradkettiges oder verzweigtes C₁-C₄-Alkoxy oder Benzyl bedeutet,
dadurch gekennzeichnet, daß man 2,3-Dichlor-nitrobenzole der Formel 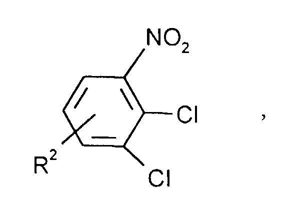 mit 0,9-2 Mol, bevorzugt 1-1,5 Mol eines Mercaptans der Formel
HS-R¹ ,
wobei
R¹ und R² die obigen Bedeutungen haben,
in Gegenwart von 1-1,2, bevorzugt 1-1,1 Äquivalenten einer Base pro Mol Mercaptan und in Gegenwart eines Phasentransferkatalysators bei einer Temperatur von 0-100°C, bevorzugt 20-80°C in wäßrigem oder wäßrigorganischem Medium umsetzt.

2. Verfahren nach Anspruch 1, dadurch gekennzeichnet, daß ein Mercaptan der Formel
HS-R¹¹ ,
eingesetzt wird, worin
R¹¹ für geradkettiges oder verzweigtes C₁-C₁₂-Alkyl, nicht substituiertes, Methyl-substituiertes oder Dimethyl-substituiertes Cyclopropyl, Cyclopentyl oder Cyclohexyl oder C₇-C₈-Aralkyl steht,
bevorzugt dadurch gekennzeichnet, daß ein Mercaptan der Formel
HS-R²¹ ,
eingesetzt wird, worin
R²¹ für geradkettiges oder verzweigtes C₁-C₄-Alkyl, Cyclopropyl, Cyclohexyl oder Benzyl steht.

3. Verfahren nach Anspruch 1, dadurch gekennzeichnet, daß ein 2,3-Dichlor-nitrobenzol der Formel 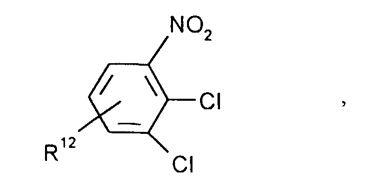 eingesetzt wird, worin
R¹² Wasserstoff, Methyl oder Methoxy bedeutet,
bevorzugt dadurch gekennzeichnet, daß 2,3-Dichlor-nitrobenzol eingesetzt wird.

4. Verfahren nach Anspruch 1, dadurch gekennzeichnet, daß eine Schmelze des Dichlor-nitrobenzols in die wäßrige Lösung eines Alkylmercaptids und eines Phasentransferkatalysators eingetragen wird.

5. Verfahren nach Anspruch 1, dadurch gekennzeichnet, daß man geschmolzenes Dichlor-nitrobenzol als Suspension in Wasser bei Gegenwart eines Phasentransferkatalysators vorlegt und eine wäßrige Alkylmercaptid-Lösung zusetzt.

6. Verfahren nach Anspruch 1, dadurch gekennzeichnet, daß die Dichlor-nitrobenzole als Lösung in einem C₁-C₆-Alkanol, C₃-C₆-Keton, C₆-C₁₂-Kohlenwasserstoff der (cyclo)aliphatischen oder aromatischen Reihe, C₁-C₈-Halogenkohlenwasserstoff der (cyclo)aliphatischen oder aromatischen Reihe oder einem offenkettigen oder cyclischen Peralkyl-carbonsäureamid mit 3 bis 8 C-Atomen eingesetzt werden.

7. Verfahren nach Anspruch 6, dadurch gekennzeichnet, daß als Lösungsmittel Methanol, Ethanol, i-Propanol, Aceton, Methylenchlorid, Chlorbenzol, Toluol, Xylol, Dimethylformamid oder N-Methyl-pyrrolidon verwendet wird.

8. Verfahren nach Anspruch 1, dadurch gekennzeichnet, daß als Phasentransferkatalysator Tetrabutylammoniumbromid oder Benzyl-trimethylammoniumchlorid verwendet wird.

9. 2-Chlor-6-nitrophenyl-alkyl-sulfane der Formel 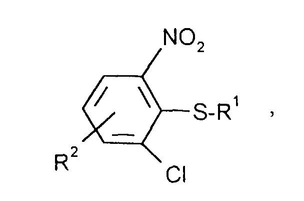 in der
R¹ für geradkettiges oder verzweigtes C₁-C₁₈-Alkyl, C₃-C₈-Cycloalkyl oder C₇-C₁₀-Aralkyl steht und
R² Wasserstoff, geradkettiges oder verzweigtes C₁-C₄-Alkyl, geradkettiges oder verzweigtes C₁-C₄-Alkoxy oder Benzyl bedeutet,
wobei für den Fall, daß R² Wasserstoff bedeutet, R¹ nicht die Bedeutung von Methyl, Ethyl oder Benzyl annimmt.

## Claims

1. Process for the preparation of 2-chloro-6- nitrophenyl alkyl sulphides of the formula 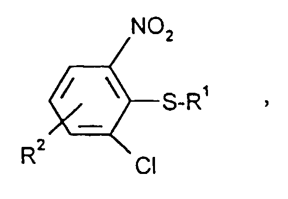 in which
R¹ is linear or branched C₁-C₁₈-alkyl, C₃-C₈-cycloalkyl or C₇-C₁₀-aralkyl and
R² is hydrogen, linear or branched C₁-C₄-alkyl, linear or branched C₁-C₄-alkoxy or benzyl,
characterized in that 2,3-dichloro-nitrobenzenes of the formula 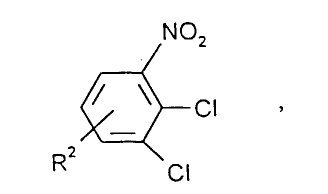 are reacted with 0.9-2 mol, preferably 1-1.5 mol, of a mercaptan of the formula
HS-R¹,
wherein
R¹ and R² are as defined above,
in the presence of 1-1.2 equivalents, preferably 1-1.1 equivalents, of a base per mole of mercaptan and in the presence of a phase transfer catalyst, at a temperature of 0-100°C, preferably 20-80°C, in an aqueous or aqueous-organic medium.

2. Process according to Claim 1, characterized in that a mercaptan of the formula
HS-R¹¹,
is used, wherein
R¹¹ is linear or branched C₁-C₁₂-alkyl, unsubstituted, methyl-substituted or dimethyl-substituted cyclopropyl, cyclopentyl or cyclohexyl, or C₇-C₈-aralkyl,
and preferably characterized in that a mercaptan of the formula
HS-R²¹,
is used, wherein
R²¹ is linear or branched C₁-C₄-alkyl, cyclopropyl, cyclohexyl or benzyl.

3. Process according to Claim 1, characterized in that a 2,3-dichloronitrobenzene of the formula 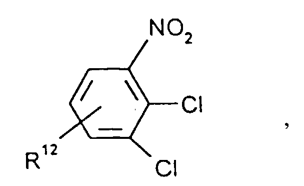 is used, wherein
R¹² is hydrogen, methyl or methoxy,
and preferably characterized in that 2,3-dichloro-nitrobenzene is used.

4. Process according to Claim 1, characterized in that a melt of the dichloro-nitrobenzene is introduced into an aqueous solution of an alkylmercaptide and a phase transfer catalyst.

5. Process according to Claim 1, characterized in that an aqueous alkylmercaptide solution is added to molten dichloro-nitrobenzene as a suspension in water in the presence of a phase transfer catalyst.

6. Process according to Claim 1, characterized in that the dichloro-nitrobenzenes are used as a solution in a C₁-C₆ alkanol, C₃-C₆ ketone, C₆-C₁₂ (cyclo)aliphatic or aromatic hydrocarbon, C₁-C₈ (cyclo)aliphatic or aromatic halogenohydrocarbon or open-chain or cyclic peralkyl-carboxamide having 3 to 8 C atoms.

7. Process according to Claim 6, characterized in that the solvent used is methanol, ethanol, i-propanol, acetone, methylene chloride, chlorobenzene, toluene, xylene, dimethylformamide or N-methyl-pyrrolidone.

8. Process according to Claim 1, characterized in that the phase transfer catalyst used is tetrabutylammonium bromide or benzyl-trimethylammonium chloride.

9. 2-Chloro-6-nitrophenyl alkyl sulphides of the formula 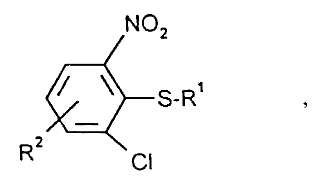 in which
R¹ is linear or branched C₁-C₁₈-alkyl, C₃-C₈-cycloalkyl or C₇-C₁₀-aralkyl and
R² is hydrogen, linear or branched C₁-C₄-alkyl, linear or branched C₁-C₄-alkoxy or benzyl,
R¹ not being methyl, ethyl or benzyl when R² is hydrogen.

## Revendications

1. Procédé pour la préparation de 2-chlore-6-nitrophényl-alkylsulfures répondant à la formule 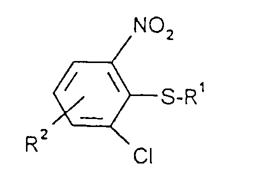 dans laquelle
R¹ représente un groupe alkyle en C₁-C₁₈ à chaîne droite ou ramifiée, un groupe cycloalkyle en C₃-C₈ ou un groupe aralkyle en C₇-C₁₀, et
R² représente un atome d'hydrogène, un groupe alkyle en C₁-C₄ à chaîne droite ou ramifiée, un groupe alcoxy en C₁-C₄ à chaîne droite ou ramifiée ou encore un groupe benzyle,
caractérisé en ce qu'on fait réagir des 2,3-dichloro-nitrobenzènes répondant à la formule 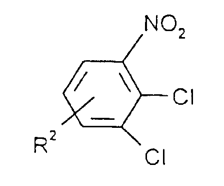 avec de 0,9 à 2 moles, de préférence de 1 à 1,5 mole d'un mercaptan répondant à la formule
HS-R¹
dans laquelle
R¹ et R² ont les significations indiquées ci-dessus,
en présence de 1-1,2, de préférence de 1-1,1 équivalent d'une base par mole de mercaptan, et en présence d'un catalyseur de transfert de phase à une température de 0-100°C, de préférence de 20-80°C, dans un milieu aqueux ou aqueux-organique.

2. Procédé selon la revendication 1, caractérisé en ce qu'on met en oeuvre un mercaptan répondant à la formule
HS-R¹¹
dans laquelle
R¹¹ représente un groupe alkyle en C₁-C₁₂ à chaîne droite ou ramifiée; un groupe cyclopropyle, un groupe cyclopentyle ou un groupe cyclohexyle non substitué ou portant un ou deux substituants méthyle; ou encore un groupe aralkyle en C₇-C₈,
de préférence caractérisé en ce qu'on met en oeuvre un mercaptan répondant à la formule
HS-R²¹
dans laquelle
R²¹ représente un groupe alkyle en C₁-C₄ à chaîne droite ou ramifiée, un groupe cyclopropyle, un groupe cyclohexyle ou un groupe benzyle.

3. Procédé selon la revendication 1, caractérisé en ce qu'on met en oeuvre un 2,3-dichloro-nitrobenzène répondant à la formule 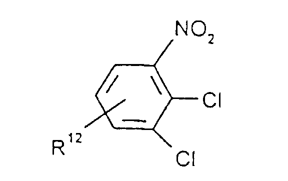 dans laquelle
R¹² représente un atome d'hydrogène, un groupe méthyle ou un groupe méthoxy,
de préférence caractérisé en ce qu'on met en oeuvre le 2,3-dichloro-nitrobenzène.

4. Procédé selon la revendication 1, caractérisé en ce qu'on introduit une masse fondue du dichloro-nitrobenzène dans la solution aqueuse d'un alkylmercaptide et d'un catalyseur de transfert de phase.

5. Procédé selon la revendication 1, caractérisé en ce qu'on dépose au préalable du dichloro-nitrobenzène porté à fusion, sous forme d'une suspension dans de l'eau, en présence d'un catalyseur de transfert de phase, et on ajoute une solution aqueuse d'alkylmercaptide.

6. Procédé selon la revendication 1, caractérisé en ce qu'on met en oeuvre les dichloronitrobenzènes sous forme d'une solution dans un alcanol en C₁-C₆, dans une cétone en C₃-C₆, dans un hydrocarbure en C₆-C₁₂ de la série des composés (cyclo)aliphatiques ou aromatiques, dans un hydrocarbure halogéné en C₁-C₈ de la série des composés (cyclo)aliphatiques ou aromatiques, ou encore dans un amide d'acide peralkyl-carboxylique à chaîne ouverte ou cyclique contenant de 3 à 8 atomes de carbone.

7. Procédé selon la revendication 6, caractérisé en ce qu'on utilise, à titre de solvants, le méthanol, l'éthanol, le i-propanol, l'acétone, le chlorure de méthylène, le chlorobenzène, le toluène, le xylène, le diméthylformamide ou la N-méthylpyrrolidone.

8. Procédé selon la revendication 1, caractérisé en ce qu'on utilise, à titre de catalyseur de transfert de phase, du bromure de tétrabutylammonium ou du chlorure de benzyltriméthylammonium.

9. 2-chloro-6-nitrophényl-alkyl-sulfures répondant à la formule 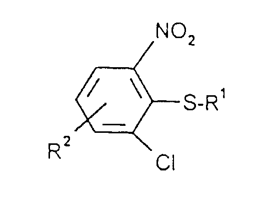 dans laquelle
R¹ représente un groupe alkyle en C₁-C₁₈ à chaîne droite ou ramifiée, un groupe cycloalkyle en C₃-C₈ ou un groupe aralkyle en C₇-C₁₀, et
R² représente un atome d'hydrogène, un groupe alkyle en C₁-C₄ à chaîne droite ou ramifiée, un groupe alcoxy en C₁-C₄ à chaîne droite ou ramifiée ou encore un groupe benzyle,
dans lesquels, pour le cas où R² représente un atome d'hydrogène, R¹ ne représente pas un groupe méthyle, un groupe éthyle ou un groupe benzyle.
